# EUROPÄISCHE PATENTSCHRIFT

(11) **EP 0 481 502 B1**
(45) Veröffentlichungstag und Bekanntmachung des Hinweises auf die Patenterteilung: **03.05.1995**
(21) Anmeldenummer: 91117772.3
(22) Anmeldetag: 17.10.1991
(51) Int. Cl.: C12N 15/13, C12P 21/08

(54) **Verfahren zur Herstellung von chimären Antikörpern**
Process for the production of chimaeric antibodies
Procédé pour la production des anticorps chimériques

(30) Priorität: 18.10.1990 DE 4033120
(43) Veröffentlichungstag der Anmeldung: 22.04.1992
(73) Patentinhaber: BOEHRINGER MANNHEIM GMBH, 68298 Mannheim (DE)
(72) Erfinder: Weidle, Ulrich Heinz, Dr. rer. nat., W-8000 München 2 (DE); Kaluza, Brigitte, Dr. rer. nat., W-8173 Bad Heilbrunn (DE)
(74) Vertreter: Huber, Bernhard, Dipl.-Chem.

(56) Entgegenhaltungen:
- EP-A- 0 368 684
- EP-A- 0 378 175
- EP-A- 0 388 914

## Beschreibung

Chimäre Antikörper, also Antikörper, bei denen die murinen oder die aus einer anderen Tierspezies stammenden konstanten Regionen durch humane konstante Regionen ersetzt sind, sind bei therapeutischer Anwendung im Menschen wesentlich weniger immunogen als die nichthumanen Antikörper und weisen damit eine dramatische Verbesserung in der therapeutischen Wirkung auf (Lo Buglio, A.F. et al, Proc. Natl. Acad. Sci. USA 86 (1989) 4220 - 4224). Zusätzlich sollten therapeutisch verwendbare Antikörper definierte Effektorfunktionen (z. B. Fixierung von Komplement, Bindung von Staphylococcus-Protein A, Kompetenz bezüglich ADCC (antibody-dependent cell-mediated cytotoxicity) und CDC (cell dependent cytotoxicity) besitzen. Reproduzierbare Effektor-Funktionen können durch Verwendung von definierten konstanten (humanen) Regionen erhalten werden.

Die Herstellung chimärer Antikörper kann nach gentechnologischen Methoden (vgl. z. B. Verhoeyen, M. und Riechmann, L.: Bio Essays 8 (1988) 74-78) erfolgen. Chimäre Antikörper sind gegen eine Reihe von Haptenen (Boulliane, N. et al., Nature 312, 643 (1984); Neuberger, M. et al., Nature 312 (1984) 604; Morrison, S.L. et al., Proc. Nat. Acad. Sci. USA 81, 6851 (1984)) und tumor-spezifische Antigene (Liu, A.Y. et al., Proc. Natl. Acad. Sci. USA 84 (1987) 3439-3443; Nishimura, Y. et al., Cancer Research 47 (1987) 999-1005) beschrieben worden.

Zur Chimärisierung von Antikörpern ist es bekannt (Shin, S.L. und Morrison, S.L.: Methods in Enzymology 178 (1989) 459-476) zuerst die Gene für die leichte und schwere Kette des entsprechenden Antikörpers aus der zugehörigen Hybridom-Linie zu isolieren. Dazu werden genomische Bibliotheken in Phagen angelegt und die gewünschten Gene durch Hybridisierung mit entsprechenden DNA-Fragmenten ermittelt. Dieses Verfahren ist außerordentlich arbeitsintensiv, da etwa 10⁶ Plaques durchgemustert werden müssen, um ein Gen zu isolieren, das nur in einer Kopie pro Maus-Chromosom vorliegt (Maniatis, T., Fritsch, E.F. and Sambrook, J.: Molecular Cloning. A Laboratory Manual. Cold Spring Harbor, NY 1982).

Da außerdem nur in einem Teil der so als positiv identifizierten Phagen die Immunglobulin-Gene in produktiv rearrangierter Form vorliegen, muß eine weitere aufwendige Charakterisierung angeschlossen werden.

Ein weiteres Verfahren ist in der EP-A 0 378 175 beschrieben. Danach werden aus der mRNA der jeweiligen Hybridom-Linie die cDNAs für die leichte und schwere Kette isoliert und die kodierenden Bereiche nach Mutagenese in entsprechende Vektoren eingesetzt. Der Nachteil dieses Verfahrens ist, daß zunächst cDNA-Klone isoliert werden müssen. Dazu muß eine cDNA Bibliothek angelegt werden, die zur Isolierung der gewünschten Klone mit entsprechenden Hybridisierungssonden durchgemustert werden muß. Dies ist zwar einfacher als das Screenen einer genomischen Bibliothek, da die messenger RNA für die leichte und schwere Ketten in Hybridom-Linien in einer Abundanz von bis zu 5 % der mRNA vorliegt. Dennoch ist die Isolierung der entsprechenden Klone zeitaufwendig. Die anschließende Sequenzierung und vor allem die Mutagenese der Isolate zur Einpassung in die vorgegebenen Expressionsvektoren im richtigen Leseraster sind ebenfalls sehr arbeitsintensiv und zeitaufwendig.

Eine Aufgabe der vorliegenden Erfindung ist es deshalb, ein Verfahren bereitzustellen, bei dem die Nachteile des Standes der Technik mindestens teilweise beseitigt sind, so daß gewünschte chimäre Antikörper auf erheblich einfachere Weise hergestellt werden können. Insbesondere sollte das Screening einer genomischen oder cDNA Bibliothek nicht mehr erforderlich sein.

Der erste Schritt für die Chimärisierung eines Antikörpers, d.h. des Austausches einer nicht-humanen konstanten Regionen (schwere und leichte Kette) durch eine andere humane konstante Region ist es, das für die gewünschte variable Region kodierende DNA-Fragment zu isolieren. Ein Gegenstand der Erfindung ist somit ein Verfahren zur Isolierung eines für die variable Region eines Antikörper-Gens kodierenden DNA-Fragments, welches dadurch gekennzeichnet ist, daß man
(a) chromosomale DNA aus einer Hybridom-Zellinie isoliert, die einen gewünschten Antikörper sezerniert,
(b) diese chromosomale DNA in Einzelstränge überführt und die einzelsträngige DNA mit zwei Oligonukleotidprimern (1) und (2) inkubiert,
   worin der Primer (1) einen Bereich enthält, der komplementär zu einer Sequenz von mindestens 10 Nukleotiden aus der 5'-untranslatierten Region des Template-Strangs des Antikörper-Gens ist und
   worin der Primer (2) einen Bereich enthält, der komplementär zu einer Sequenz von mindestens 10 Nukleotiden aus der 3'-untranslatierten Region der jeweiligen J-Region des Nicht-Template-Strangs des Antikörper-Gens ist,
(c) DNA-Polymerase und Desoxyribonukleosidtriphosphate zugibt, so daß eine Elongation der Primer stattfindet, wobei ein doppelsträngiges DNA-Fragment in dem Teilbereich des Antikörper-Gens gebildet wird, der zwischen den Primern liegt,
(d) die so gebildete doppelsträngige DNA zu Einzelsträngen denaturiert, die einzelsträngige DNA wieder mit zwei Oligonukleotidprimern (1) und (2) zusammenbringt, die Primer elongiert, so daß doppelsträngige DNA entsteht, und
(e) die Prozedur von Schritt (d) gegebenenfalls mehrfach wiederholt.

Ein Antikörper ist aus mindestens zwei Polypeptidketten zusammengesetzt, der schweren (H) und der leichen (L) Kette. Jede dieser Ketten besteht aus einer konstanten und einer variablen Region, wobei die variablen Regionen eines Antikörpers seine Antigen-Spezifität bestimmen. Die Chimärisierung des Antikörpers bedeutet den Austausch einer vorzugsweise nicht-humanen konstanten Region durch eine andere vorzugsweise humane konstante Region. Durch das erfindungsgemäße Verfahren ist es nun auf einfache Weise möglich, ein DNA-Fragment zu isolieren, das für die variable Region einer bestimmten Kette eines Antikörpers kodiert. Dieses DNA-Fragment kann nun zur Expression der Kette eines chimären Antikörpers verwendet werden. Hierfür ist es erforderlich, die variablen Regionen sowohl der H-Kette als auch der L-Kette zu isolieren.

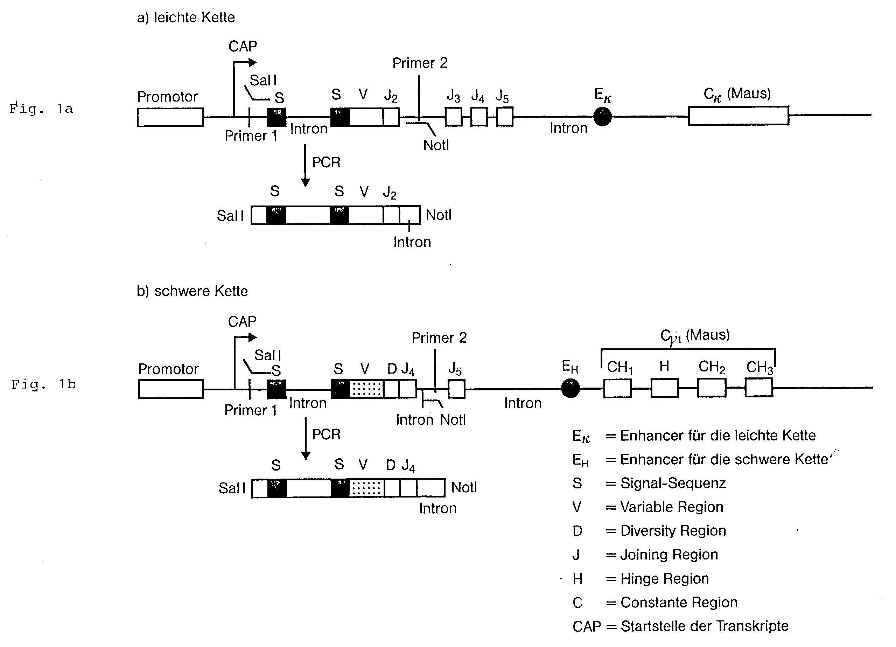

Ein DNA-Fragment, das für die variable Region der L-Kette kodiert und durch das erfindungsgemäße Verfahren isoliert wird, enthält die aus Figur 1a ersichtlichen und zwischen den Primern (1) und (2) liegenden Bereiche. Ein solches DNA-Fragment enthält die folgenden Bereiche eines Antikörper-Gens: einen Teil der 5'-untranslatierten Region, der zu Primer (1) komplementär ist, die durch ein Intron unterbrochene Signalsequenz, die variable (V) Region und die für das jeweilige Gen verwendete Joining-(J)Region. Am 3'-Ende des Fragments liegt der nicht translatierte Teil der J-Region mit einer zu Primer (2) komplementären Sequenz. In Figur 1a ist ein durch das erfindungsgemäße Verfahren nach Amplifikation mit den Primern (1) und (2) erhaltenes DNA-Fragment ersichtlich.

Ein DNA-Fragment, das für die variable Region einer H-Kette kodiert, enthält im wesentlichen dieselben funktionellen Elemente mit dem Unterschied, daß die variable Region der H-Kette zwischen der V-Region und der J-Region noch eine Diversity(D)-Region enthält. Auch hier ist aus Figur 1b ersichtlich, daß das zwischen den Primern (1) und (2) liegende DNA-Fragment durch das erfindungsgemäße Verfahren nach Amplifikation mit den Primern (1) und (2) erhalten wird.

Voraussetzung für die Durchführung des erfindungsgemäßen Verfahrens ist die Synthese von geeigneten Primern (1) und (2). Dafür sind bestimmte Kenntnisse über die DNA-Sequenz der leichten und schweren Kette des zu chimärisierenden Antikörpers erforderlich, d.h., man muß die zu den Primern (1) und (2) komplementäre Sequenz auf dem Antikörper-Gen im Bereich der 5'-untranslatierten Region bzw. in der untranslatierten Region (3') der verwendeten J-Region kennen. Diese Information kann direkt oder indirekt durch Sequenzierung der mRNA der Antikörper-Gene oder einer aus der mRNA hergestellten cDNA erhalten werden, wie weiter unten im Detail beschrieben wird.

Die Primer (1) und (2) enthalten einen mindestens 10 Nukleotide langen, zur chromosomalen DNA des Antikörpers komplementären Bereich. Vorzugsweise ist dieser Bereich 10 bis 50 Nukleotide lang, um gute Hybridisierung an die einzelsträngige DNA zu ergeben. Primer (1) enthält Sequenzen aus dem 5'-untranslatierten Bereich des Antikörper-Gens, wobei die genaue Lokalisation in diesem Bereich an sich beliebig ist. Primer (1) kann auch teilweise Nukleotide aus der Signalsequenz, die in 3'-Richtung an die 5'-untranslatierte Region anschließt, enthalten. Vorzugsweise liegt Primer (1) jedoch nicht 5'-seitig der Transkriptionsstartstelle des Antikörper-Gens.

Primer (2) enthält Sequenzen aus der 3'-untranslatierten Region der J-Region des Antikörper-Gens, d.h. Sequenzen 3'-seitig der im jeweiligen Antikörper-Gen translatierten J-Region. Wird z.B. in einem Antikörper-Gen die J₂-Region verwendet, kann Primer (2) Sequenzen aus den Bereichen von J₂, J₃, J₄ oder J₅ enthalten, wobei die genaue Lokalisation von Primer (2) in diesem Bereich an sich beliebig ist. In einer bevorzugten Ausführungsform enthält der Primer (2) Teile der Sequenz der an das Intron anschließenden Sequenz der jeweiligen J-Region.

Durch die Orientierung der beiden Primer ist sichergestellt, daß bei Schritt (c) des erfindungsgemäßen Verfahrens die Elongation der Primer in Richtung aufeinander zu erfolgt, so daß ein DNA-Fragment entsteht, dessen doppelsträngiger Bereich die variable Region des Antikörper-Gens umfaßt. Dieses Fragment wird erfindungsgemäß durch die an sich bekannte PCR-Reaktion in mehreren Zyklen, vorzugsweise 10 bis 30 Zyklen amplifiziert. Als Endprodukt des erfindungsgemäßen Verfahrens erhält man ein DNA-Fragment, an dessen Enden sich die Primer (1) und (2) befinden.

Vorzugsweise enthalten die Primer (1) und (2) 5'-seitig von dem Bereich, der zum Antikörper-Gen komplementär ist, noch eine Restriktionsendonuklease-Schnittstelle, die natürlich zum Antikörper-Gen nicht komplementär sein muß. Vorzugsweise enthalten Primer (1) und (2) Schnittstellen für Restriktionsendonukleasen mit einer Erkennungssequenz von 6 bis 8 Nukleotiden, besonders bevorzugt von solchen Enzymen, deren Erkennungsstellen sich selten auf eukaryontischer DNA befinden (z.B. SalI, NotI). Besonders bevorzugt sind die Restriktionsendonuklease-Schnittstellen von Primer (1) und (2) verschieden. Zwischen der komplementären Sequenz und der Erkennungssequenz für die Restriktionsendonuklease können die Primer beliebige Sequenzen (von 1 bis 30 Nukleotide) enthalten.

Weiterhin ist es günstig, wenn die Primer (1) und (2) 5'-seitig von der Restriktionsendonuklease-Schnittstelle noch einen Überhang von mindestens zwei Nukleotiden enthalten, um nach Isolierung des DNA-Fragments zwei Spaltungen mit Restriktionsendonukleasen an den Schnittstellen der Primer (1) und (2) durchführen zu können. Auf diese Weise kann das erfindungsgemäß isolierte DNA-Fragment leichter in einen Vektor kloniert werden.

Die Elongation der Primer bei Durchführung des erfindungsgemäßen Verfahrens erfolgt durch die dem Fachmann an sich bekannte PCR-Reaktion (EP-A 0 201 184). Dabei verwendet man als DNA-Polymerase vorzugsweise Taq-Polymerase und denaturiert die doppelsträngige DNA nach jedem Zyklus durch Erhitzen.

Voraussetzung für das erfindungsgemäße Verfahren ist zunächst die Ermittlung von Sequenz-Informationen des zu chimärisierenden Antikörper-Gens. Ohne diese Information ist die Konstruktion der Primer (1) und (2) nicht möglich.

Die Sequenzierung von Teilbereichen des Antikörper-Gens, deren Sequenz für das erfindungsgemäße Verfahren bekannt sein muß, geschieht durch folgendes Verfahren:

Aus der Hybridomazellinie des zu chimärisierenden Antikörpers, dessen 5'untranslatierte Sequenz der messenger RNA für die leichte und schwere Kette bestimmt werden soll und für den festgestellt werden soll, welche J-Regionen für die leichte bzw. schwere Kette benutzt werden, wird mRNA isoliert und ein Primer, welcher zum 5'-Ende der konstanten Region komplementär ist, mit reverser Transkriptase elongiert. Die Sequenz der konstanten Regionen ist literaturbekannt (Kabat, E.A., Wu, T.T., Reid-Miller, M., Perry, H.M. und Gottesman, K.S.; Sequenzces of Proteins of Immunological Interest, 4th Edition, US Department of Health and Human Services (1987), National Institut of Health) und die im jeweiligen Antikörper-Gen vorhandene konstante Region kann immunologisch leicht bestimmt werden.

An den 3'-Enden des entstandenen cDNA-Primärstrangs werden mittels terminaler Desoxynukleotidyltransferase 10 - 30 definierte Oligonukleotide (z. B. Oligo-G) anpolymerisiert. Durch Zugabe von zwei Primern (3) und (4) (erster Primer (3) Oligo-C, vorzugsweise mit einer ersten Restriktionsendonuklease-Schnittstelle; zweiter Primer (4) aus der literaturbekannten Sequenz der konstanten Region des zu chimärisierenden Antikörpers (s.o.) wird eine Amplifikationsreaktion durchgeführt, die amplifizierte cDNA subkloniert und in den erforderlichen Bereichen (5'untranslatierte Region, J-Region) sequenziert. Auf diese Weise erhält man die Sequenz-Information für Primer (1). Die Sequenz-Information für Primer (2) erhält man indirekt dadurch, daß man die im jeweiligen Antikörper-Gen translatierte J-Region bestimmt. Dies ist möglich, da die genomische Organisation der J Regionen für die leichte Kette (K) der Maus mit den zugehörigen Intron-Sequenzen beschrieben ist (Max, E.E. et al., J. Biol. Chem. 256 (1981) 5116-5120). Die äquivalente Information für die kodierenden und Intron-Sequenzen für die J-Regionen der schweren Ketten der Maus ist beschrieben in: Sakano, H. et al.: Nature 286 (1980) 676-683. Die Durchführung der Amplifikationsreaktion erfolgt wie in Saiki, R.K. et al.: Science 230 (1985) 1350 oder EP-A 0 201 184 beschrieben.

Alternativ dazu kann die Sequenz durch direkte Sequenzierung der mRNA für die leichte und die schwere Kette erhalten werden (Hamlyn, P.H. et al, Cell 15 (1978) 1067 - 1075). Hierbei werden polyA⁺ RNA isoliert und synthetische Oligonukleotide, welche zu den 5'-Regionen der bekannten konstanten Regionen für die leichte und die schwere Kette hybridisieren, mit reverser Transkriptase verlängert. Die Sequenz der cDNA wird mit der Methode von Sanger und Coulson (J. Mol. Biol. 94 (1975) 441 - 448) bestimmt. Hierbei werden 2', 3'-Dideoxyribonukleotide für den Kettenabbruch verwendet.

Die durch das erfindungsgemäße Verfahren isolierten Gen-Abschnitte von variablen Antikörper-Regionen können direkt zur Expression von Antikörper-Fragmenten (Fab, F(ab)₂) verwendet werden oder an andere Gene (z.B. für Enzyme, Toxine und für konstante Antikörper-Regionen) gekoppelt werden. Vorzugsweise erzeugt man chimäre Antikörper. Außer IgG 1 (wie im Beispiel) können durch Wahl der jeweiligen konstanten Region chimärisierte Antikörper der Isotypen IgG 2, IgG 3, IgG 4, IgM, IgA, IgD und IgE konstruiert werden.

Die Isolierung eines für die variable Region eines Antikörper-Gens kodierenden DNA-Fragments ist ein erster Schritt zur Chimärisierung von Antikörpern. Der nächste Schritt ist ein Verfahren zur Herstellung von chimären Antikörper-Genen, wobei man ein nach dem erfindungsgemäßen Verfahren isoliertes DNA-Fragment in einen Vektor einführt, der ein DNA-Fragment enthält, das für eine gewünschte konstante Region eines Antikörper-Gens kodiert, wobei ein aus variabler und konstanter Region in operativer Verknüpfung bestehendes chimäres Antikörper-Gen entsteht. Dabei verwendet man vorzugsweise einen Vektor, der den Genabschnitt für eine humane konstante Region enthält. Möchte man den Genabschnitt für die L-Kette eines chimären Antikörpers exprimieren, verwendet man einen Vektor, der den Genabschnitt für die konstante Region einer L-Kette enthält. Möchte man ein Gen für die H-Kette eines chimären Antikörpers exprimieren, so verwendet man einen Vektor, der den Genabschnitt für die konstante Region einer H-Kette enthält. Vorzugsweise verwendet man einen Vektor, der für eine spätere Expression des Antikörper-Gens notwendige Elemente, insbesondere einen Promotor und eine Polyadenylierungsstelle in richtiger Lokalisation enthält.

Bevorzugte Basisvektoren sind pSV2-neo (Southern, P. and Berg, P. J., Mol. Appl. Genet. 1 (1982) 327 - 341 oder pSV2-Eco gpt (Mulligan, R.C. and Berg, P., Proc. Natl. Acad. Sci. USA 78 (1981) 2072 - 2076). Bevorzugte Promotoren sind Immunglobulin Promotoren, der frühe oder späte Promotor von Simian Virus 40, der murine oder humane Cytomegalie-Virus Promotor oder der Metallothionein Promotor der Maus oder des Menschen. Der Expressionsvektor enthält vorzugsweise zusätzlich Enhancer-Elemente, welche in lymphoiden Zellen aktiv sind, z. B. von Immunglobulin Genen, von Simian Virus 40, Polyoma, murinem oder humanem Cytomegalie-Virus.

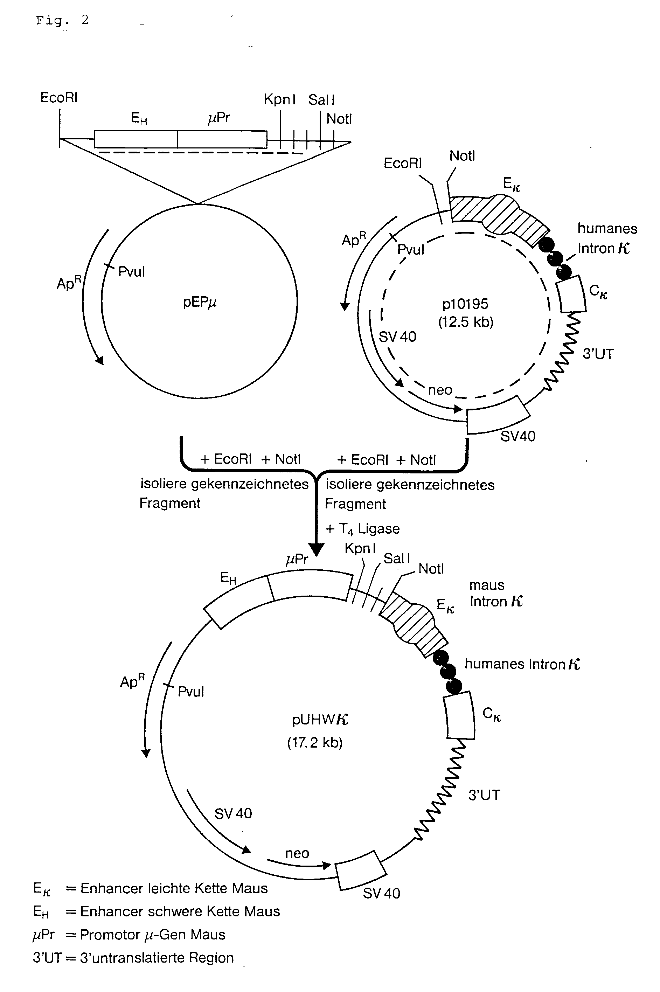

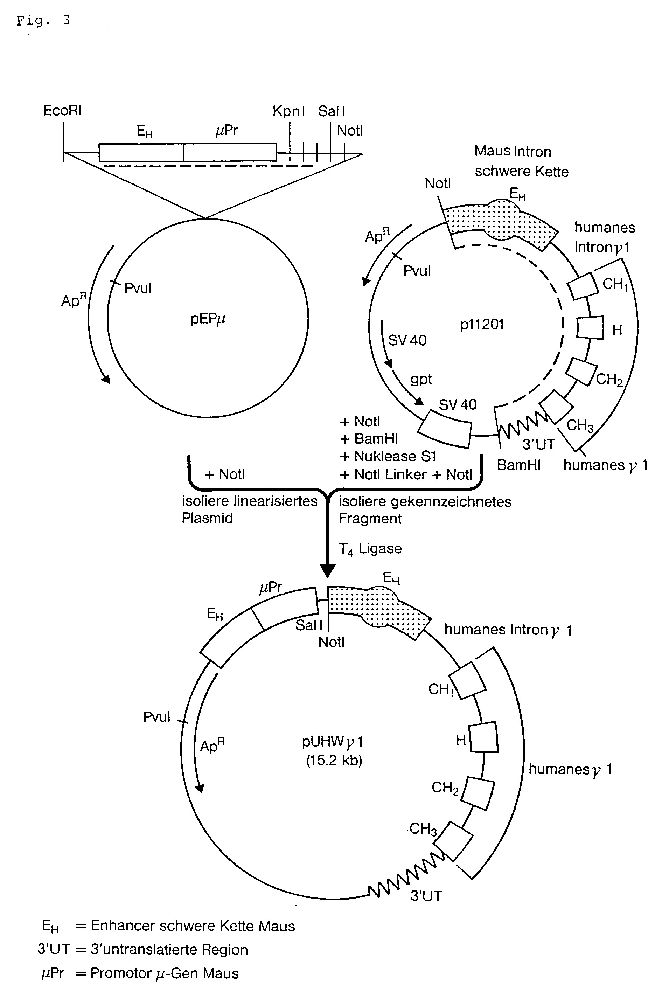

Besonders bevorzugte Basisvektoren sind die in den Figuren 2 und 3 dargestellten Vektoren pUHWk (enthält die humane Konstante K Region der L-Kette) bzw. pUHWγ1 (enthält die humane konstante Region).

Bei Klonierung der variablen Region der L-Kette des von der Zellinie 15-1/P3/14 (ECACC 90090705) sekretierten Antikörpers gegen das CD4-Antigen erhält man so den Vektor pUHW-CD4K (DSM 6156). Bei Klonierung der variablen Region der H-Kette des von der Zelllinie 15-1/P3/14 (ECACC 90090705) sekretierten Antikörpers gegen das CD4-Antigen erhält man so den Vektor pUHW-CD4γ1 (DSM 6155).

Ein weiteres Beispiel für eine Antikörper-produzierende Zelllinie, aus der durch das erfindungsgemäße Verfahren die für die variablen Regionen des Antikörpers kodierenden DNA-Fragmente gewonnen werden konnten, ist die Zellinie 3.10-1/5B10 (ECACC 90090702). Der von dieser Zellinie sekretierte Antikörper erkennt ein anderes Epitop auf dem CD4-Antigen.

Auf analoge Weise lassen sich jedoch auf gleiche Weise variable Regionen, die durch das erfindungsgemäße Verfahren isoliert wurden und von beliebigen Antikörpern stammen, in geeignete Basisvektoren klonieren.

Mit dem erfindungsgemäßen Verfahren gelingt es, genomische Fragmente von leichten und schweren Ketten von Antikörpern, die damit das Intron der Signalsequenz und Teilbereiche des Introns der J-Sequenz enthalten, auf einfache Weise zu isolieren. Mit Vektoren, welche diese genomischen Fragmente enthalten, wird eine wesentlich höhere Ausbeute an chimären Antikörpern erhalten, als wenn nur die cDNA des chimären Antikörpers zur Expression verwendet wird.

Analog läßt sich das erfindungsgemäße Verfahren anwenden zur Durchführung von Isotyp-Switching bei humanen Hybridom-Linien. Hier sind die Antikörper fast aller auftretenden Klone vom Isotyp IgM. Da diesem Isotyp aber entscheidende Effektor-Funktionen, wie z.B. ADCC (antibody-dependent cell-mediated cytotoxicity) fehlen und das pentamere IgM-Molekül zur Multimerisierung und Aggregierung neigt, ist hier die Durchführung eines Isotyp-Switches (definiertes Verändern der konstanten Kette des Antikörpers) nach der erfindungsgemäßen Methode vorteilhaft, um Bindungs-Eigenschaften und Effektor-Funktionen der jeweiligen Isotypen zu evaluieren. Die bekannten Verfahren zur Durchführung eines Isotyp-Switches mit zellbiologischen Methoden (Rothman, P. et al., Mol. Cell. Biol. 10 (1990) 1672-1679) durch Interleukin 4, Lipopolysaccharid (LPS), oder LPS in Kombination mit Interleukin 4 sind hier wegen der außerordentlich tiefen Frequenz des erwarteten Ereignisses wenig erfolgreich. Da die Sequenz der J-Regionen für leichte und schwere Ketten von menschlichen Immunglobulin-Genen bekannt ist (P.A. Hieter, J.v. Maizel Jr., P. Leder, J.Biol.Chem. Vol. 257, Nr. 3 (1982), 1516-1522; J.V. Ravetch, U. Siebenlist, S. Korsmeyer, T. Waldmann, P. Leder, Cell 27 (1981), 583-591), kann die Konstruktion der für das erfindungsgemäße Verfahren wesentlichen Primer (1) und (2) so erfolgen, wie oben beschrieben.

Weiterhin ein Gegenstand der vorliegenden Erfindung ist ein Verfahren zur Gewinnung von chimären Antikörpern, wobei man eine geeignete Zelle mit zwei gemäß einem erfindungsgemäßen Verfahren hergestellten Vektoren transfiziert, wobei einer dieser Vektoren das Gen für die H-Kette eines chimären Antikorpers enthält und der andere ein Gen für die L-Kette dieses chimären Antikörpers enthält, die transfizierten Zellen auf stabile Transfektanten selektioniert und den aktiven Antikörper aus dem Zellmedium auf übliche Weise gewinnt.

Geeignete Zellen im Sinne der Erfindung sind vorzugsweise sogenannte Non-Immunglobulin-Producer-Hybridomzellinien, das sind Hybridomlinien, die keine Antikörper sezernieren. Die Transfektion in diese Zellen kann auf eine dem Fachmann an sich bekannte Weise erfolgen. Beispiele für solche HybridomLinien sind etwa Sp2/0-Ag14 (Ochi, A. et al, Proc. Natl. Acad. Sci. USA 80 (1983) 6351 - 6355 oder P3X63-Ag8.653 (Kearney, J.F. et al, J. Immunol. 123 (1979) 1548 - 1550). Die Selektion stabiler Transfektanten erfolgt nach Lenz, H. und Weidle, U.H.: Gene 87 (1990) 213-218.

Die Erfindung wird durch die folgenden Beispiele in Verbindung mit den Figuren weiter verdeutlicht. Es zeigen:
- Fig. 1a:: die Gewinnung eines für die variable Region einer L-Kette kodierenden DNA-Fragments
- Fig. 1b:: die Gewinnung eines für die variable Region einer H-Kette kodierenden DNA-Fragments
- Fig. 2:: die Konstruktion eines Expressions-Basisvektors für ein chimäres Antikörper-Gen der L-Kette
- Fig. 3:: die Konstruktion eines Expressions-Basisvektors für ein chimäres Antikörper-Gen der H-Kette

Die Antikörper-produzierenden Zellinien 15-1/P3/14 (ECACC 90090705) und 3.10-1/5B10 (ECACC 90090702) wurden bei European Collection of Animal Cell Cultures, GB-Salisbury, Wiltshire SP4 OJG hinterlegt. Die Vektoren pUHW-CD4k (DSM 6156) und pUHW-CD4γ1 (DSM 6155) wurden bei Deutsche Sammlung von Mikroorganismen und Zellkulturen GmbH, Mascheroder Weg 1b, D-3300 Braunschweig hinterlegt.

### Beispiel 1

### Sequenzierung der mRNA eines zu chimärisierenden Antikörpers

Vorbemerkung: In dem hier beschriebenen Beispiel wird eine murine Hybridom-Linie verwendet, welche Antikörper (Typ IgG1) sezerniert, und ein Epitop auf der extrazellulären Region des T-Lymphozyten Rezeptors (human) CD4 erkennen. Die leichte Kette ist vom Typ k (kappa), die schwere Kette vom Typ γ1. Die Hybridom-Linie trägt die Bezeichnung 15-1/P3/14. Dieser Antikörper hat therapeutisches Potential bei der Behandlung von Autoimmun-Erkrankungen, wie z.B. rheumatoider Arthritis.

### a) Sequenzierung der mRNA und von Primern für die PCR

Aus 10⁶ Zellen der Hybridom-Linie 15-1/P3/14 wurde messenger RNA aus Gesamt-RNA durch Chromatographie an Oligo-dT-Cellulose isoliert wie beschrieben (Maniatis et al., Molecular Cloning. A Laboratory Manual. Cold Spring Harbor Laboratory, Cold Spring Harbor, NY, 1982, Seite 283).

Die anschließende Sequenzierung der mRNA für die leichte und die schwere Kette erfolgt durch Primer-Elongation mit reverser Transkriptase wie beschrieben in Fred Sablitzky and Klaus Rajewski, EMBO Journal 3, 3005-3017 (1984) und Hamlyn, P.H. et al, Cell 15 (1978) 1067 - 1075.

Die cDNA-Synthese wurde in einem Reaktionsvolumen von 10 »l durchgeführt.

Bedingungen: 0,1 »g mRNA, 0,05 »g Primer, 0,5 »l reverse Transkriptase (9000 U/»l), Deoxynukleosidtriphosphate 50 »mol/l, ³²P markiertes Nukleotidtriphosphat 4 »mol/l.

Jeder Reaktionsansatz enthielt zusätzlich ein anderes Dideoxynukleosidtriphosphat (ddATP, ddTTP, ddGTP oder ddCTP, Konzentration 10 »mol/l). Als Puffer wurde verwendet: 50 mmol/l Tris-HCl, pH 8,3, 60 mmol/l NaCl, 20 mmol/l Dithiothreitol, 6 mmol/l MgCl₂. Die Reaktionsgemische wurden bei 37°C für 30 Minuten inkubiert und mit 10 »l Formamid in 10 mmol/l EDTA und Bromphenol-Blau und Xylencyanol FF abgestoppt. Aliquots wurden auf ein Sequenzier-Gel aufgetragen und die Sequenz der cDNA nach Sanger, F., Nicklen, S. und Coulson, A.R., Proc. Natl. Acad. Sci. USA 74 (1977), 5463 - 5467 durch Autoradiographie bestimmt.
- Verwendete Primer:: komplementär zu einem Bereich der konstanten Region (beschrieben in EMBO-J. Rajewski, Supra)

a) k-Kette: 5'TGCAGCATCAGCCC3'
b) γ1-Kette: 5'GGCCAGTGGATAGAC3'
Die Sequenzen ergaben Informationen über:
a) die 5'-untranslatierten Regionen der mRNAs für die leichte und die schwere Kette, so daß Primer (1) konzipiert werden kann.
b) die in der leichten, bzw. schweren Kette jeweils verwendete J-Region, so daß man Informationen zur Konstruktion von Primer (2) erhält. Es zeigte sich für den Fall des eingesetzten Antikörpers, daß die leichte Kette eine J2-Region, die schwere Kette eine J4-Region besitzt.
Im folgenden sind die 5'-untranslatierten Sequenzen der schweren und leichten Kette angegeben. Aus diesen Sequenzen können geeignete Primer (1) (siehe Beispiel 2.1 a) und c)) ausgewählt werden.
5'-Untranslatierte Region (leichte Kette):
5' - AAGACTCAGCCTGGACATGATG - 3' Initiationskodon
5'-Untranslatierte Region (schwere Kette): 
5′ - TGACCAGTTAGTCTTAAGGCACCACTGAGC - 3′
CCAAGTCTTAGACATCATG
Initiationskodon

### Beispiel 2

### Amplifikation der genomischen DNA der variablen Regionen der leichten und schweren Kette des zu chimärisierenden Antikörpers

Mit der Information aus Beispiel 1 über die Sequenz des zu chimärisierenden Antikörpers war es möglich, Primer zu konzipieren, welche zu Intron-Sequenzen der jeweiligen J-Region für die leichte und die schwere Kette komplementär sind (Max, E.E. et al., J. Biol. Chem. 256 (1981) 5116-5120 = für die J-Region der leichten Kette und Sakano, H. et al.: Nature 286 (1980) 676-683 für die J-Region der schweren Kette) und zusätzlich eine Erkennungsstelle für eine Restriktionsendonuklease enthalten.

### 2.1 Verwendete Primer:

a) leichte Kette 5'Primer (Primer 1): untranslatierte Region + SalI Stelle = 35 mer 
5′ GATC GTCGAC AAGACTCAGCCTGGACATGATGTCC 3′
Über- untranslatierte Region k
hang SalI
 Restriktionsendonuklease -Schnittstelle
b) leichte Kette 3'Primer (Primer 2): J₂-Intron + NotI Stelle = 35 mer 
5' ATCA GCGGCCGC ACTTAACAAGGTTAGACTTAGTG 3'
Überhang Intron J2 Kappa
NotI
 Restriktionsendonuklease -Schnittstelle
c) 5' Primer (Primer 1) schwere Kette: untranslatierte Region + SalI Stelle = 35 mer 
5' CATA GTCGAC GTTAGTCTTAAGGCACCACTGAGCC 3'
Über- untranslatierte Region γ1
hang SalI
 Restriktionsendonuklease-Schnittstelle
d) 3'Primer (Primer 2) schwere Kette: Intron J4 + NotI Stelle = 38 mer 
5′ GAGC | GCGGCCGC | TTAAAAATAAAGACCTGGAGAGGCCA 3′
Über- Intron J4 für γ1
hang NotI
 Restriktionsendonuklease-Schnittstelle

### 2.2 Amplifikation der relevanten VJ (leichte Kette) und VDJ-Regionen (schwere Kette) durch PCR.

Die zu amplifizierenden Bereiche für die leichte und schwere Kette sind Fig. 1 dargestellt. Für die Amplifikation der L-Kette wurden die Primer a und b und für die Amplifikation der H-Kette die Primer c und d verwendet.

Chromosomale DNA wurde aus der Hybridom-Linie 15-1/P3/14 nach Standard-Methoden isoliert (Maniatis, T. et al., Molecular Cloning. A Laboratory Manual. Cold Spring Harbor Laboratory, Cold Spring Harbor, NY 1982, Seite 283).
Die PCR Reaktion wurde in einem Perkin-Elmer Cetus Thermo-Cycler durchgeführt (Zyklenzahl: 26).

### Reaktionsbedingungen:

10 mmol/l TRIS · HCl, pH 8.3, 50 mmol/l KCl, 1.5 mmol/l MgCl₂, 0,1 % (w/v) Gelatine, 1 »g chromosomale DNA, 5' und 3' Primer je 1 »mol/l, dATP, dGTP, dCTP, dTTP jeweils 200 »mol/l, Taq Polymerase (Perkin Elmer) 2.5 Units. Reaktionsvolumen: 100 »l. Es wurde mit 100 »l "Mineral oil light" (Sigma) überschichtet.
- Reaktionsdauer pro Zyklus:: 2 min
- Reaktionstemperatur pro Zyklus:: 72 °C
Vor dem 1. Zyklus wird die Reaktionsmischung 5 min bei 92 °C, anschließend 2 min bei 55 °C und dann 2 min bei 72 °C gehalten. Vor den darauf folgenden Zyklen wurde die Reaktionsmischung jeweils 2 min bei den oben genannten Temperaturen gehalten. Als Kontrolle wurde in einem weiteren Ansatz ein 2,5 kb Fragment aus dem Maus-Urokinase Gen amplifiziert. Nach der Reaktion wurde ein Aliquot (10 % des Reaktionsansatzes) entnommen und auf einem 1 % Agarose-Gel (mit 0,5 »g/ml Ethidium-Bromid) untersucht.

Es zeigte sich im Fall der leichten Kette Amplifikation eines Fragments von 560 bp, im Fall der schweren Kette wurde ein 650 bp Fragment amplifiziert.

Zur Isolierung der amplifizierten Fragmente wurde das restliche Reaktions-Gemisch phenolisiert, die wässrige Phase mit Ethanol präzipitiert, die DNA in entsprechendem Puffer gelöst und anschließend mit den Restriktions-Endonukleasen SalI und NotI nachgespalten. Die amplifizierten Fragmente wurden auf einem 0,8 %igen niedrig-schmelzenden Agarose-Gel aufgereinigt und die Ausbeute bestimmt.

Zur Sequenzierung wurde ein Aliquot in den kommerziell erhältlichen Vektor pUC19 (Yanisch-Perron et al., Gene 33 (1985) 103-109) einligiert, bei dem die unikate HindIII-Stelle im Polylinker in eine NotI-Stelle umgewandelt worden war (nach Bluntieren der HindIII-Schnittstelle durch Nuklease S1 und anschließendes Einligieren eines NotI-Linkers). Nach Einligieren der amplifizierten SalI-NotI Fragmente in den mit SalI und NotI geschnittenen modifizierten pUC Vektor wurden Ampicillin-resistente Kolonien (50 »g/ml) isoliert und die rekombinanten Plasmide durch Restriktions-Analyse charakterisiert.

Jeweils 5 Plasmide mit den amplifizierten SalI-NotI Fragmenten für die leichte und die schwere Kette (welche jeweils bei der Restriktions-Enzym Analyse identische Banden-Muster zeigten) wurden sequenziert nach Sanger, F. et al., loc.cit. Abgesehen von dem in der Signal-Sequenz bei der leichten und schweren Kette auftretenden Intron zeigte sich Übereinstimmung mit den zuvor ermittelten cDNA Sequenzen.

### Beispiel 3

### Herstellung von Expressionsvektoren zur Expression der leichten und schweren Ketten des chimären Antikörpers

Die nach Beispiel 2 hergestellten und amplifizierten VJ- bzw. VDJ-Regionen des Antikörpers 15-1/P3/14 wurden als SalI-NotI Fragmente in die mit SalI und NotI geschnittenen Kassetten-Expressions-Vektoren für die chimärisierten Antikörper einligiert.

### 3.1 Konstruktion des Kassetten-Vektors pUHWk

In einer Vorkonstruktion wurde die unikate HindIII-Stelle in Plasmid pUC18 (Yanisch-Perron, C. et al., Gene 33 (1985) 103-109) in eine NotI-Stelle umgewandelt. Dazu wurde pUC18 mit HindIII linearisiert, die überhängenden Enden mit Nuklease S1 bluntiert, ein NotI-Linker anligiert, mit NotI nachgeschnitten, das linearisierte Plasmid auf einem niedrig-schmelzenden Agarose-Gel aufgereinigt, religiert und nach Transfektion in E. coli HB 101 Ampicillin resistente Kolonien auf Agar-Platten selektioniert. Die gewünschte Rekombinante wurde durch Analyse mit Restriktions-Endonukleasen charakterisiert. Das entsprechende Plasmid wird als pUC18 (NotI) bezeichnet. Zur Konstruktion von pEP» wurde pUC18 (NotI) in der multiplen Klonierungs-Stelle mit den Enzymen EcoRI und KpnI geschnitten und ein 2 kb EcoRI-KpnI Fragment aus Plasmid pIgγEcogpt (Weidle et al., Gene 60 (1987) 205-216) einligiert. Das beschriebene Fragment enthält den Enhancer eines »Gens als 0.6 kb Fragment (Banerji et al., Cell 33 (1983) 729-740) und das 1.4 kb Promotor Fragment eines Maus »-Gens (Neuberger, M: EMBO J. 2 (1983) 1373-1378) hintereinander angeordnet (Fig. 2).

Für die weitere Konstruktion wird Plasmid p10195 (hergestellt nach EP-A 0 378 175) benötigt. Das Plasmid enthält ein chimäres Intron aus Intron-Sequenzen der leichten Kette der Maus und des Menschen, die konstante Region der k-Kette des Menschen sowie eine Expressions-Kassette für die Phosphotransferase neo. pUHWk wurde erhalten, nachdem das 2 kb EcoRI-NotI Fragment (enthaltend schwere Kette Enhancer-Promotor Kombination) aus pEP» in das mit EcoRI und NotI geöffnete Plasmid p10195 einligiert worden war (Fig. 2).

Die in Beispiel 2 beschriebenen isolierten amplifizierten genomischen VJ Fragmente mit überhängenden SalI und NotI Schnittstellen werden gerichtet in das mit SalI und NotI geöffnete Plasmid pUHWk zur Erstellung von Expressions-Vektoren für die chimärisierten leichten Ketten des jeweiligen Antikörpers einligiert.
Das im hier beschriebenen Beispiel erhaltene Expressions-Plasmid für die chimärisierte leichte Kette des Antikörpers 15-1/P3/14 wird mit pUHW-CD4k bezeichnet.

### 3.2 Konstruktion des Kassetten-Vektors pUHWγ1

Das hierbei verwendete Plasmid p11201 ist beschrieben in der EP-A 0 378 175. Es enthält ein chimäres Intron aus Intron-Sequenzen der schweren Immunglobulin-Ketten der Maus und des Menschen sowie die genomische Struktur der konstanten Region des humanen γ1 Gens. p11201 wurde mit NotI und BamHI geschnitten, und die Enden mit Nuklease S1 bluntiert. Anschließend wurden NotI Linker (GCGGCCGC) anligiert (T4 Ligase) und das in der Abbildung gekennzeichnete Fragment auf einem niedrig-schmelzenden Agarose-Gel isoliert. Das beschriebene Fragment wurde in die unikate NotI-Stelle von Plasmid pEP» einligiert, in E. coli HB 101 transfektiert und Ampicillin-resistente (50 »g/ml) Kolonien auf Agar-Platten isoliert. Dabei wurde zunächst Plasmid pUHW*γ1 erhalten. Dieses Plasmid enthält die genomische humane γ1 constante Region in der gewünschten Orientierung bezüglich der Immunglobulin-Promotor-Enhancer Kombination, besitzt aber zwei NotI Stellen, was die gerichtete Klonierung der durch PCR erhaltenen VDJ SalI-NotI Fragmente erschwert (Fig. 3).

Zur Eliminierung der unerwünschten NotI Stelle wurde pUHW*γ1 partiell mit NotI gespalten, die überhängenden Enden mit Nuklease S1 bluntiert, das Fragment auf einem niedrigschmelzenden Agarose-Gel aufgereinigt, religiert, in E. coli HB 101 transfektiert und Ampicillin-resistente Kolonien (50 »g/ml) auf Agar-Platten selektioniert. Durch Analyse mit Restriktionsendonukleasen wurde das Plasmid ermittelt, bei dem die unerwünschte NotI-Stelle nach der 3'-untranslatierten Region der konstanten Region deletiert worden war. Das resultierende Plasmid wird als pUHWγ1 bezeichnet (s. Fig. 3). Die durch PCR amplifizierten VDJ-Regionen enthaltenden SalI-NotI Fragmente von schweren Ketten von Immunglobulin-Genen werden gerichtet in das mit SalI und NotI geschnittene Plasmid pUHWγ1 zur Erstellung von Expressions-Vektoren für die chimärisierte schwere Kette eines Antikörpers einkloniert. Der im Falle des Antikörpers 15-1/P3/14 erhaltene Expressions-Vektor für die chimärisierte schwere Kette wird als pUHW-CD4γ1 bezeichnet.

### Beispiel 4

### Erstellung von permanenten Zell-Linien, welche konstitutiv chimärisierte CD4 Antikörper sezernieren.

Die Plasmide pUHW-CD4k und pUHW-CD4γ1 wurden durch Elektroporation (Gene Pulser von Biorad) in die Immunglobulin Non-Producer Hybridom Zell-Linie Sp2/0-Ag14 (ATCC CRL 1581) (Ochi, A. et al., Proc. Natl. Acad. Sci. USA 80 (1983) 6351-6355) transfektiert, wie beschrieben in Nucleic Acids Res. 15 (1987) 1311-1326 und Bio Techniques 6 (1988) 742-751. Pro Puls wurden 10 »g Plasmid und 10⁶ Zellen eingesetzt.

Nach Abzentrifugieren werden die Zellen mit kaltem HeBs-Puffer (20 mmol/l HEPES, pH 7.05, 137 mmol/l NaCl, 5 mmol/l KCl, 0,7 mmol/l Na₂HPO₄, 6 mmol/l Dextrose) gewaschen, mit HeBs-Puffer resuspendiert, auf eine Konzentration von 10⁶ Zellen pro ml eingestellt und auf Eis gestellt. Nach Plasmid-Zugabe wurde gepulst (Bedingungen: Kapazität 160 »F und 200 bis 260 V). Die Zellen werden nach dem Pulsen ca. 10 Minuten auf Eis gehalten und anschließend in Medium (RPMI 1640, 10 % foetales Kälberserum, 2 mmol/L Glutamin, 1 mmol/l Natriumpyruvat, 0,1 mol/l nicht essentielle Aminosäuren) bei 37 °C inkubiert. Die Selektion stabiler Transformanten erfolgte durch Wachstum auf G 418-haltigem Medium bei einer Konzentration von 800 »g G418/ml Medium.

Stabile Transformanten wurden durch "limited dilution" isoliert und konnten nach etwa 14 Tagen identifiziert werden. Sie sezernierten den chimärisierten CD4 Antikörper (IgG1) in hoher Ausbeute.

### Beispiel 5

### Nachweis von rekonstituiertem Anti CD4 in Überständen von Transfektoma-Linien

Alle Arbeitsschritte werden bei Raumtemperatur durchgeführt.

Die Vertiefungen einer Mikrotiterplatte werden mit je 0,2 ml einer Lösung von 2,5 »g/ml Schaf-Anti-human-Fcγ-IgG in 0,2 mol/l Bicarbonat/Carbonat-Puffer, pH 9,5 beschickt und 1 h inkubiert.

Nach Aussaugen werden je 0,3 ml einer Lösung von 1 % Gelatine-Hydrolysat in 50 mmol/l HEPES-Puffer, 0,1 mol/l NaCl, pH 7,0 (Inkubationspuffer) zur Absättigung restlicher Proteinbindungsstellen in die Vertiefungen gefüllt und 30 min inkubiert.

Nach Aussaugen werden je 0,2 ml Eichproben bzw. Verdünnungen (in Inkubationspuffer) von Kulturüberständen in die Vertiefungen pipettiert und 1 h inkubiert.

Nach Aussaugen werden je 0,2 ml einer Lösung (in Inkubationspuffer) eines Konjugats aus monoklonalem Antikörper (MAK) und Peroxidase (Herstellung nach US Patent No. 4,657,853, Example 3) und 25 ng/ml rekombinantem CD4-Antigen (American Bio-Technologies Inc.) in die Vertiefungen gefüllt und erneut 1 h inkubiert.

Nach dreimaligem Waschen mit je 0,3 ml 50 mmol/l HEPES-Puffer, 0,15 mol/l NaCl, 0,1 % Synperonic® F68 (Detergenz) werden je 0,2 ml ABTS-Substrat-Lösung (9,1 mmol/l ABTS® (2,2'-Azino-di-[3-ethyl- benzthiazolin-sulfonsäure(6)]-diammoniumsalz), 100 mmol/l Phosphat-Citrat-Puffer pH 5,0, 1,47 mmol/l Natriumperborat) pipettiert und nach 30 - 45 min Inkubation die Extinktionen in einem ELISA-Reader bei 405 nm gemessen.

### Beispiel 6

Das in den Beispielen 1 bis 3 beschriebene Verfahren wurde analog zur Chimärisierung eines zweiten gegen das CD4-Antigen gerichteten Antikörpers angewandt, der von der Zellinie 3.10-1/5B10 (ECACC 90090702) sekretiert wird. Dieser Antikörper erkennt ein vom Antikörper 15-1/P3/14 verschiedenes Epitop auf dem extrazellulären Anteil von humanem CD4.

Die verwendeten Primer für die Isolierung der VJ-Region für die leichte Kette (Kappa) hatten die folgende Sequenz:
a) 5'-Primer für die leichte Kette (Primer 1): untranslatierte Region + SalI-Stelle = 34 mer 
5' ATCA GTCGAC GCAGCTGCCAGGAGCCTAGAAGCA
Über- untranslatierte Region k
hang SalI
 Restriktionsendonuklease-Schnittstelle
b) 3'-Primer für die leichte Kette (Primer 2): J₁-Intron + NotI-Stelle = 35 mer 
5' ATCA GCGGCCGC ACAACGGAAGAAAGAGACTTTGG
[?]Überhang Intron J1 k
NotI
 Restriktionsendonuklease-Schnittstelle
c) 5'-Primer für die schwere Kette (Primer 1): untranslatierte Region + SalI-Stelle = 38 mer 
5' CATA | GTCGAC | GTCCCTGAACACACTGACTCTAACCATG
Über- | | untranslatierte Region γ1
hang SalI
 Restriktionsendonuklease-Schnittstelle
d) 3'-Primer für die schwere Kette (Primer 2): J₃-Intron + NotI-Stelle = 35 mer 
ATCA GCGGCCGC GGTTGAATCTTGATTCCCGTTTG
Über- Intron J3 für γ1
hang NotI
 Restriktionsendonuklease-Schnittstelle
   Mit den beschriebenen Primern wurden die relevanten variablen Regionen (VJ-Region bei der leichten Kette bzw. VDJ-Region bei der schweren Kette) gemäß Beispiel 3 durch PCR amplifiziert und wie in Beispiel 4 beschrieben in die entsprechenden Expressionsbasisvektoren pUHWk bzw. pUHWγ1 einkloniert.

Im folgenden sind Teilbereiche der untranslatierten Regionen der leichten Kette und der schweren Kette der für den Antikörper 3.10-1/5B10 kodierenden Gene angegeben. Aus diesen Sequenzen können geeignete 5'-Primer (Primer 1) ausgewählt werden (siehe z.B. oben a) und c)).

Untranslatierte Region der leichten Kette (Kappa-Kette): 
5′ - GCAGCTGCCAGGAGCCTAGAAGCATCC
TCTCATCTAGTTCTCAGAGATG - 3′
Initiationskodon
Untranslatierte Region der schweren Kette (γ1-Kette):
5' - ACAGTCCCTGAACACACTGACTCTAACCATG - 3'
Initiationskodon

## Patentansprüche

1. Verfahren zum Isolieren eines für die variable Region eines Antikörper-Gens kodierenden DNA-Fragments,
**dadurch gekennzeichnet,**
daß man
(a) chromosomale DNA aus einer Hybridom-Zellinie isoliert, die einen gewünschten Antikörper sezerniert,
(b) diese chromosomale DNA in Einzelstränge überführt und die einzelsträngige DNA mit zwei Oligonukleotidprimern (1) und (2) inkubiert,
worin der Primer (1) einen Bereich enthält, der komplementär zu einer Sequenz von mindestens 10 Nukleotiden aus der 5'-untranslatierten Region des Template-Strangs des Antikörper-Gens ist und worin der Primer (2) einen Bereich enthält, der komplementär zu einer Sequenz von mindestens 10 Nukleotiden aus der 3'-untranslatierten Region der jeweiligen J-Region des Nicht-Template-Strangs des Antikörper-Gens ist,
(c) DNA-Polymerase und Desoxyribonukleosidtriphosphate zugibt, so daß eine Elongation der Primer stattfindet, wobei ein doppelsträngiges DNA-Fragment in dem Teilbereich des Antikörper-Gens gebildet wird, der zwischen den Primern liegt,
(d) die so gebildete doppelsträngige DNA zu Einzelsträngen denaturiert, die einzelsträngige DNA wieder mit zwei Oligonukleotidprimern (1) und (2) zusammenbringt, die Primer elongiert, so daß doppelsträngige DNA entsteht, und
(e) die Prozedur von Schritt (d) gegebenenfalls mehrfach wiederholt.

2. Verfahren nach Anspruch 1,
**dadurch gekennzeichnet,**
daß man Primer (1) und (2) verwendet, die 5'-seitig vom komplementären Bereich noch eine Restriktionsendonukleasen-Schnittstelle enthalten.

3. Verfahren zum Herstellen von chimären Antikörper-Genen,
**dadurch gekennzeichnet,**
daß man ein gemäß einem Verfahren nach einem der Ansprüche 1 oder 2 isoliertes DNA-Fragment in einen Basisvektor einführt, der ein DNA-Fragment enthält, das für die konstante Region der leichten oder schweren Kette eines Antikörper-Gens kodiert, wobei ein aus variabler und konstanter Region in operativer Verknüpfung bestehendes chimäres Antikörper-Gen entsteht.

4. Verfahren nach Anspruch 3,
**dadurch gekennzeichnet,**
daß man einen Basisvektor verwendet, der den Genabschnitt für eine humane konstante Region eines Antikörpers enthält.

5. Verfahren nach Anspruch 3 oder 4,
**dadurch gekennzeichnet**,
daß man einen Basisvektor verwendet, der einen Promotor und eine Polyadenylierungsstelle enthält.

6. Verfahren nach einem der Ansprüche 3-5,
**dadurch gekennzeichnet**,
daß man einen Basisvektor verwendet, der in lymphoiden Zellen aktive Enhancer-Elemente enthält.

7. Verfahren zur Gewinnung von chimären Antikörpern,
**dadurch gekennzeichnet,**
daß man eine geeignete Zelle mit zwei gemäß einem Verfahren nach einem der Ansprüche 3 bis 6 hergestellten Vektoren transfiziert, wobei einer davon ein Gen für die schwere Kette eines chimären Antikörpers enthält und der andere ein Gen für die leichte Kette dieses chimären Antikörpers enthält, auf stabile Transfektanten selektioniert und den aktiven Antikörper auf übliche Weise gewinnt.

8. Verfahren nach Anspruch 7,
**dadurch gekennzeichnet,**
daß man die beiden Vektoren in eine Non-Immunglobulin-Producer-Hybridomzellinie einführt.

9. Verfahren nach Anspruch 7 oder 8,
**dadurch gekennzeichnet,**
daß man einen chimärisierten Antikörper gewinnt, der von der Hybridom-Linie 15-1/P3/14 (ECACC 90090705) sezerniert wird.

10. Verfahren nach Anspruch 9,
**dadurch gekennzeichnet,**
daß man als Vektor für die schwere Kette des chimären Antikörpers pUHW CD4-γ1 (DSM 6155) und als Vektor für die leichte Kette des chimären Antikörpers pUHW CD4-k (DSM 6156) verwendet.

11. Verfahren nach Anspruch 7 oder 8,
**dadurch gekennzeichnet,**
daß man einen chimärisierten Antikörper gewinnt, der von der Hybridomlinie 3.10-1/5B10 (ECACC 90090702) sezerniert wird.

12. Verfahren zur Herstellung eines Arzneimittels
**dadurch gekennzeichnet,**
daß man einen oder mehrere chimäre Antikörper, die gemäß einem Verfahren nach einem der Ansprüche 7 bis 11 gewonnen wurden, sowie gegebenenfalls bekannte Hilfs-, Zusatz-, Träger-und Füllstoffe formuliert.

## Claims

1. Process for the isolation of a DNA fragment coding for the variable region of an antibody gene, **wherein**
(a) chromosomal DNA is isolated from a hybridoma cell line which secretes a desired antibody,
(b) this chromosomal DNA is converted into single strands and the single-stranded DNA is incubated with two oligonucleotide primers (1) and (2),
in which primer (1) contains a region which is complementary to a sequence of at least 10 nucleotides from the 5' untranslated region of the template strand of the antibody gene and
in which primer (2) contains a region which is complementary to a sequence of at least 10 nucleotides from the 3' untranslated region of the respective J region of the non-template strand of the antibody gene,
(c) DNA polymerase and deoxyribonucleoside triphosphates are added so that an elongation of the primers takes place whereby a double stranded DNA fragment is formed in the partial region of the antibody gene which lies between the primers,
(d) the double-stranded DNA formed in this way is denatured to single strands, the single-stranded DNA is again brought together with two oligonucleotide primers (1) and (2), the primers are elongated so that double-stranded DNA forms and
(e) the procedure of step (d) is repeated several times if desired.

2. Process as claimed in claim 1,
**wherein**
primers (1) and (2) are used which contain another restriction endonuclease cleavage site on the 5' side of the complementary region.

3. Process for the production of chimeric antibody genes,
**wherein**
a DNA fragment isolated according to a process as claimed in one of the claims 1 or 2 is introduced into a base vector which contains a DNA fragment which codes for the constant region of the light or the heavy chain of an antibody gene whereby a chimeric antibody gene is formed consisting of the variable and constant region in operational linkage.

4. Process as claimed in claim 3,
**wherein**
an base vector is used which contains the gene section for a human constant region of an antibody.

5. Process as claimed in claim 3 or 4,
**wherein**
a base vector is used which contains a promoter and a polyadenylation site.

6. Process as claimed in one of the claims 3-5,
**wherein**
a base vector is used which contains enhancer elements active in lymphoid cells.

7. Process for the production of chimeric antibodies,
**wherein**
a suitable cell is transfected with two vectors produced according to a process as claimed in one of the claims 3 to 6 whereby one of them contains a gene for the heavy chain of a chimeric antibody and the other contains a gene for the light chain of this chimeric antibody, one selects for stable transfectants and the active antibody is isolated in the usual way.

8. Process as claimed in claim 7,
**wherein**
both the vectors are introduced into a non-immunoglobulin producer hybridoma cell line.

9. Process as claimed in claims 7 or 8,
**wherein**
a chimerized antibody is isolated which is secreted by the hybridoma line 15-1/P3/14 (ECACC 90090705).

10. Process as claimed in claim 9,
**wherein**
pUHW CD4-γ1 (DSM 6155) is used as the vector for the heavy chain of the chimeric antibody and pUHW CD4-k (DSM 6156) is used as the vector for the light chain of the chimeric antibody.

11. Process as claimed in claims 7 or 8,
**wherein**
a chimerized antibody is isolated which is secreted by the hybridoma line 3.10-1/5B10 (ECACC 90090702).

12. Process for the production of a pharmaceutical preparation,
**wherein**
one or several chimeric antibodies are formulated,
which were obtained according to a process as claimed in one of the claims 7 to 11, as well as if desired known auxiliary agents, additives, carrier substances and filling agents.

## Revendications

1. Procédé pour isoler un fragment d'ADN codant la région variaole d'un gène d'anticorps, caractérisé en ce que
(a) on isole l'ADN chromosomique d'une lignée cellulaire d'hybridome qui sécrète un anticorps souhaité,
(b) on convertit cet ADN chromosomique en simples brins et on incube l'ADN simple brin avec deux amorces oligonucléotidiques (1) et (2),
parmi lesquelles l'amorce (1) contient un domaine qui est complémentaire d'une séquence d'au moins 10 nucléotides de la région non traduite en 5' du brin matrice du gène d'anticorps et l'amorce (2) contient un domaine qui est complémentaire d'une séquence d'au moins 10 nucléotides de la région non traduite en 3' de la région J correspondante du brin non matrice du gène d'anticorps,
(c) on ajoute une ADN polymérase et des désoxyribonucléosides triphosphates de sorte qu'il se produit une élongation des amorces et qu'il se forme un fragment d'ADN double brin dans le domaine partiel du gène d'anticorps qui est situé entre les amorces,
(d) l'ADN double brin ainsi formé est dénaturé en simples brins, l'ADN simple brin est de nouveau mis en présence de deux amorces oligonucléotidiques (1) et (2), les amorces subissent une élongation, de sorte qu'il se forme un ADN double brin, et
(e) le processus de l'étape (d) est éventuellement répété plusieurs fois.

2. Procédé selon la revendication 1, caractérisé en ce que l'on utilise des amorces (1) et (2) qui, du côté 5' du domaine complémentaire, contiennent encore un site de coupure pour une endonucléase de restriction.

3. Procédé de préparation de gènes d'anticorps chimériques, caractérisé en ce que l'on introduit un fragment d'ADN isolé par un procédé selon l'une des revendications 1 ou 2 dans un vecteur de base qui contient un fragment d'ADN qui code la région constante de la chaîne légère ou lourde d'un gène d'anticorps, de sorte qu'il se forme un gène d'anticorps chimérique consistant en une région variable et une région constante en liaison fonctionnelle.

4. Procédé selon la revendication 3, caractérisé en ce que l'on utilise un vecteur de base qui contient le segment de gène pour une région constante humaine d'un anticorps.

5. Procédé selon la revendication 3 ou 4, caractérisé en ce que l'on utilise un vecteur de base qui contient un promoteur et un site de polyadénylation.

6. Procédé selon l'une des revendications 3 à 5, caractérisé en ce que l'on utilise un vecteur de base qui contient des éléments stimulateurs actifs dans les cellules lymphoïdes.

7. Procédé d'obtention d'anticorps chimériques, caractérisé en ce que l'on transfecte une cellule appropriée avec deux vecteurs préparés par un procédé selon l'une des revendications 3 à 6, l'un d'eux contenant un gène pour la chaîne lourde d'un anticorps chimérique et l'autre contenant un gène pour la chaîne légère de cet anticorps chimérique, on opère une sélection pour les transfectants stables et on obtient de manière habituelle l'anticorps actif.

8. Procédé selon la revendication 7, caractérisé en ce que l'on introduit les deux vecteurs dans une lignée cellulaire d'hybridome non productrice d'immunoglobulines.

9. Procédé selon la revendication 7 ou 8, caractérisé en ce que l'on obtient un anticorps chimérisé qui est sécrété par la lignée d'hybridome 15-1/P3/14 (ECACC 90090705).

10. Procédé selon la revendication 9, caractérisé en ce que l'on utilise comme vecteur pour la chaîne lourde de l'anticorps chimérique pUHW CD4-γ1 (DSM 6155) et comme vecteur pour la chaîne légère de l'anticorps chimérique pUHW CD4-k (DSM 6156).

11. Procédé selon la revendication 7 ou 8, caractérisé en ce que l'on obtient un anticorps chimérisé qui est sécrété par la lignée d'hybridome 3.10-1/5B10 (ECACC 90090702).

12. Procédé de préparation d'un médicament caractérisé en ce que l'on formule un ou plusieurs anticorps chimériques qui ont été obtenus par un procédé selon l'une des revendications 7 à 11 et éventuellement des adjuvants, additifs, véhicules et charges connus.
